# EUROPEAN PATENT APPLICATION

(11) **EP 3 586 875 A1**
(43) Date of publication of application: **01.01.2020**
(21) Application number: 18179145.0
(22) Date of filing: 21.06.2018
(51) Int. Cl.: A61K 47/10, A61K 47/26

(54) **STABILIZATION OF POLYSORBATE**

(71) Applicant: Lonza Limited, 3930 Visp (CH)
(72) Inventor: SCHMIDT, Nina Ariane, 79540 Loerrach (DE); JAHN, Michael, 79539 Loerrach (DE); MAHLER, Hanns-Christian, 79541 Loerrach (DE)
(74) Representative: Page, Richard Mark Donald

(57) **Abstract**

The invention relates to compositions comprising polysorbate and antioxidants such as butylated hydroxytoluene and butylated hydroxyanisole, and to a method for reducing the oxidative degradation of polysorbate in a composition by the use of antioxidants such as butylated hydroxytoluene and butylated hydroxyanisole.

## Description

### FIELD OF THE INVENTION

The invention relates to compositions comprising polysorbate and antioxidants such as butylated hydroxytoluene and butylated hydroxyanisole, and to a method for reducing the oxidative degradation of polysorbate in a composition by the use of antioxidants such as butylated hydroxytoluene and butylated hydroxyanisole.

### BACKGROUND OF THE INVENTION

Polysorbates are a class of non-ionic surfactants which are used widely in protein pharmaceuticals to stabilize the proteins against interface-induced aggregation and to minimize surface adsorption of proteins. Around 70% of marketed monoclonal antibody formulations contain either polysorbate 20 or 80. Polysorbates are also used as wetting agents or emulsifiers in food products, for example, polysorbate is added at a concentration of up to 0.5% (v/v) to ice cream to make it smoother and to increase its resistance to melting. Polysorbates are further used as emulsifiers in cosmetics such as shampoo, after shave, body lotion and make-up, in particular to solubilize oils into water-based products.
Polysorbates are amphiphilic, non-ionic surfactants composed of a hydrophilic polyoxyethylene (POE) sorbitan head group and a hydrophobic fatty acid tail. Commercially available polysorbates are chemically diverse mixtures containing mainly sorbitan POE fatty acid esters. The prevalent use of polysorbates is due to their high hydrophile-lipophile balance (HLB) values, low critical micelle concentration (CMC) values and thus very efficient surface activity at low concentrations. In the case of protein stabilization, polysorbates interact at interfaces in competition with a protein, thereby minimizing surface-induced denaturation and aggregation and surface adsorption of proteins.
Unfortunately, polysorbates are prone to degradation by auto-oxidation and hydrolysis. Oxidation typically occurs via two mechanisms: (1) the autoxidation of the ethylene oxide group and (2) radical oxidation at the site of unsaturation (Kishore et al., 2011, J. Pharm. Sci. 100:721-731). Hydrolytic polysorbate degradation produces predominantly fatty acids and oxidative polysorbate degradation produces more diverse degradation products including peroxides, aldehydes, acids, ketones, n-alkanes, fatty acid esters, and other degradation products (Ravuri et al., 2011, Pharm. Res. 28: 1194-1210).
In particular, when polysorbate-containing aqueous solutions are exposed to air there is a significant risk of oxidative degradation of polysorbates.
The prior art has shown that polysorbate oxidation under oxidative stress conditions can be somewhat mitigated in protein formulations by co-formulating with antioxidants. For example, WO 2011/089062 discloses a liquid pharmaceutical formulation comprising a protein, a surfactant and at least one antioxidant selected from the group of radical scavengers, chelators or chain terminators. However, relatively high ratios of antioxidants to polysorbates are required.
There is a need to more effectively reduce the oxidative degradation of polysorbate in compositions containing polysorbate to avoid loss of its surfactant properties over time. This will result in more stable polysorbate-containing compositions during processing, during long-term storage, and during their application, which in turn will lengthen the shelf-life of such compositions.
The present invention is based on the finding that the antioxidants butylated hydroxytoluene (BHT) and butylated hydroxyanisole (BHA) are highly effective in reducing the oxidative degradation of polysorbate. Remarkably, this effect is achieved by BHT or BHA alone. Advantageously, this effect is also achieved with low amounts of antioxidant relative to polysorbate, i.e. when polysorbate is present in a large excess compared to BHT or BHA.
It will be appreciated that the stabilization of polysorbate by relatively low amounts of BHT or BHA, according to the invention, renders polysorbate-containing compositions advantageous for pharmaceutical applications.

The abbreviations used in the present specification are as follows:
API - Active pharmaceutical ingredients
4HNE - 4-hydroxynonenal
BHA - butylated hydroxyanisole
BHT - butylated hydroxytoluene
ESI - Electrospray ionization
FFA - Free fatty acids
FLD - Fluorescence detector
FMA - Fluorescence micelle assay
FOX assay - ferrous oxidation xylenol orange assay
HPLC - High performance liquid chromatography
HS-GC-MS - Headspace gas chromatography mass spectrometry
LC-UV-MS - Liquid chromatography with ultraviolet/visible and mass spectrometry
NPN - N-phenyl-1-naphtylamine
ppb - Parts per billion, based on weight per volume of composition
PS - Polysorbate
PS20 - Polysorbate 20
PS80 - Polysorbate 80
RL - Reporting limit
TIC - Total ion current
WR - Working reagent.

Where an indefinite or definite article is used when referring to a singular noun, e.g. "a", "an" or "the", this includes a plural of that noun unless something else is specifically stated.

### SUMMARY OF THE INVENTION

Subject of the invention is a composition COMP comprising a polysorbate POLSORB and an antioxidant ANTOX selected from the group consisting of butylated hydroxytoluene, butylated hydroxyanisole, and mixtures thereof.

### BRIEF DESCRIPTION OF THE DRAWINGS

Figure 1A measures by FOX assay the peroxide concentrations in samples of 10% PS20-containing aqueous solutions under the T0 condition (stored at -20°C for 7 weeks), H₂O₂ condition (stored at 25°C for 7 weeks in the presence of 10% H₂O₂ based on the molar ratio of used antioxidant) and Air condition (stored at 40°C for 7 weeks and exposed to air). The PS20-containing aqueous solutions are either unstabilized, or they contain 0.02% (w/v) BHT or BHA.
Figure 1B measures by FOX assay the peroxide concentrations in samples of 10% PS80-containing aqueous solutions under the T0, H₂O₂ and Air conditions. The PS80-containing aqueous solutions are either unstabilized, or they contain 0.02% (w/v) BHT or BHA.
Figure 2 measures by LC-UV-MS the 4HNE levels in 10% PS80-containing aqueous solutions under the T0 and Air conditions. The PS80-containing aqueous solutions are either unstabilized, or they contain 0.02% (w/v) BHT or BHA.
Figure 3 measures by HPLC-FMA the polysorbate concentrations in 10% PS20-containing aqueous solutions under the T0 and Air conditions. The PS20-containing aqueous solutions are either unstabilized, or they contain 0.02% (w/v) BHT or BHA. Percent is weight of polysorbate per volume of the composition.
Figure 4 measures by HPLC-FMA the polysorbate concentrations in 10% PS80-containing aqueous solutions under the T0 and Air conditions (left side) and in 0.1% PS80-containing aqueous solutions under the T0 and H₂O₂ conditions (right side). The 10% PS80-containing aqueous solutions are either unstabilized, or they contain 0.02% (w/v) BHT or BHA. The 0.1% PS80-containing aqueous solutions are either unstabilized, or they contain 0.0002% (w/v) BHT or BHA. Percent is weight of polysorbate per volume of the composition.
Figure 5A measures by LC-MS the lauric acid levels (in ppb) in 10% PS20-containing aqueous solutions under the T0 and Air conditions. The PS20-containing aqueous solutions are either unstabilized, or they contain 0.02% (w/v) BHT or BHA.
Figure 5B measures by LC-MS the myristic acid levels (in ppb) in 10% PS20-containing aqueous solutions under the T0 and Air conditions. The PS20-containing aqueous solutions are either unstabilized, or they contain 0.02% (w/v) BHT or BHA.
Figure 5C measures by LC-MS the palmitic acid levels (in ppb) in 10% PS20-containing aqueous solutions under the T0 and Air conditions. The PS20-containing aqueous solutions are either unstabilized, or they contain 0.02% (w/v) BHT or BHA.
Figure 6A measures by LC-MS the palmitic acid levels (in ppb) in 10% PS80-containing aqueous solutions under the T0 and Air conditions. The PS80-containing aqueous solutions are either unstabilized, or they contain 0.02% (w/v) BHT or BHA.
Figure 6B measures by LC-MS stearic acid levels (in ppb) in 10% PS80-containing aqueous solutions under the T0 and Air conditions. The PS80-containing aqueous solutions are either unstabilized, or they contain 0.02% (w/v) BHT or BHA.
Figure 6C measures by LC-MS oleic acid levels (in ppb) in 10% PS80-containing aqueous solutions under the T0 and Air conditions. The PS80-containing aqueous solutions are either unstabilized, or they contain 0.02% (w/v) BHT or BHA.

### DETAILED DESCRIPTION

Preferably, POLSORB is selected from the group consisting of polysorbate 20, polysorbate 21, polysorbate 40, polysorbate 60, polysorbate 61, polysorbate 65, polysorbate 80, polysorbate 81, polysorbate 85, and polysorbate 120, and mixtures thereof;
more preferably, POLSORB is polysorbate 20 or polysorbate 80.

COMP can comprise besides ANTOX and POLSORB further components FURTHCOMP such as typically found in pharmaceutical formulations, such as API, solvents such as water, carriers, excipients, stabilizers, preservatives or other components. Preferably, FURTHCOMP are physiologically acceptable components.
API can be any API known to the skilled person, such as small molecule APIs, large molecule APIs, such as proteins, antibodies, antibody-drug conjugates, peptides, RNA, DNA, oligonucleotides, or polynucleotides.
As used herein, the term "antibody" may be understood in the broadest sense as any immunoglobulin (Ig) that enables binding to its epitope. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system.
The antibody can be a "monoclonal antibody" or "mAb", which is an antibody obtained from a population of substantially homogeneous antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.
Preferably COMP is an aqueous composition.
In case COMP is an aqueous composition, it can comprise a buffer. The buffer can, for example, be used to stabilize the pH of the solution.
In case COMP is an aqueous composition, COMP can have a pH of 2 to 12, preferably of 2.5 to 10, more preferably of 3.5 to 8, and even more preferably of 4 to 7. In one embodiment, COMP can comprise PS20 and have a pH of 4 to 6.5. In another embodiment, COMP can comprise PS80 and have a pH of 4 to 7.
In a particular embodiment, COMP can comprise 10% PS20 and have a pH of 4 to 6,
in another particular embodiment, COMP can comprise 0.1% PS20 and have a pH of 5 to 6.5,
in another particular embodiment, COMP can comprise 10% PS80 and have a pH of 4 to 5,
in another particular embodiment, COMP can comprise 0.1% PS80 and have a pH of 5 to 7.
Suitable buffers are well known in the art. Such buffers are for example histidine-buffers, citrate-buffers, succinate-buffers, acetate-buffers, phosphate-buffers, and mixtures thereof. Preferred buffers are citrate, L-histidine or mixtures of L-histidine and L-histidine hydrochloride. Independently from the buffer used, the pH can be adjusted with an acid or a base known in the art, e.g. hydrochloric acid, acetic acid, phosphoric acid, sulfuric acid, citric acid, sodium hydroxide or potassium hydroxide.

COMP may be aqueous solutions, suspensions, emulsions, dispersions, creams, ointments, gels, lotions, shampoos, soaps, tooth paste, or sprays. Preferably, COMP are aqueous solutions.

Carriers can be any carrier known to the skilled person in the art such as for example aqueous liquids; dextrose solutions; glycerol solutions; microemulsions; nanoparticles; liposomal suspensions; oils, including those of petroleum, animal, vegetable or synthetic origin, such as peanut oil, soybean oil, mineral oil, and sesame oil; isopropyl alcohol, gaseous fluorocarbons, ethyl alcohol, polyvinyl pyrrolidone, propylene glycol, a gel-producing material, stearyl alcohol, stearic acid, spermaceti, sorbitan monooleate, and methylcellulose; as well as combinations thereof.
Excipients can be any excipient known to the skilled person in the art such as for example starch, glucose, lactose, sucrose, gelatin, silica gel, sodium stearate, glycerol, glycerol monostearate, talc, sodium chloride, propylene, glycol, and ethanol; as well as combinations thereof.
Stabilizers can be any stabilizers known to the skilled person in the art such as for example amino acids; ascorbic acid; surfactants such as poloxamer; polyhydric sugar alcohols, preferably trihydric or higher sugar alcohols, such as glycerin, erythritol, arabitol, xylitol, sorbitol and mannitol; as well as combinations thereof.
Preservatives can be any preservative known to the skilled person in the art, such as for example octadecyldimethylbenzyl ammonium chloride, hexamethonium chloride, benzalkonium chloride, benzethonium chloride, phenol, butyl or benzyl alcohol, alkyl parabens such as methyl or propyl paraben, catechol, resorcinol, cyclohexanol, 3-pentanol, and m-cresol.
Yet other components in COMP can be hydrophilic polymers such as polyethylene glycol (PEG); monosaccharides; disaccharides; including mannose and trehalose; oligosaccharides, polysaccharides, and other carbohydrates including dextrins or dextrans; chelating agents such as EDTA; salt-forming counter-ions such as sodium; metal complexes (e.g. Zn-protein complexes); and fatty acid esters, fatty acid ethers or sugar esters.

Preferably COMP comprises from 0.001 to 99.99999%, more preferably from 0.01 to 75%, even more preferably from 0.01 to 50%, especially from 0.001 to 30 %, more especially from 0.01 to 20%, even more especially from 0.05 to 15%, in particular from 0.075 to 12.5%, of POLSORB; the percentages are weight of POLSORB/volume of COMP.
Preferably COMP comprises from 0.001 to 99.99999%, more preferably from 0.001 to 99.999%, even more preferably from 0.01 to 99.99%, especially from 0.01 to 99.9%, more especially 0.01 to 75%, even more especially from 0.01 to 50%, in particular from 0.001 to 30 %, more in particular from 0.01 to 20%, even more in particular from 0.05 to 15%, much more in particular from 0.075 to 12.5%, of POLSORB; the percentages are weight percent based on the weight of COMP.
In case that COMP consists of POLSORB and ANTOX, then preferably COMP comprises from 80 to 99.99999%, more preferably from 90 to 99.999%, even more preferably from 95 to 99.99%, especially from 97.5 to 99.9%, more especially from 99 to 99.9%, of POLSORB; the percentages are weight percent based on the weight of COMP; the amounts of POLSORB and ANTOX adding up to 100% by weight based on the weight of COMP.

Preferably COMP comprises from 0.00001 to 20%, more preferably from 0.001 to 10%, even more preferably from 0.01 to 5%, especially from 0.1 to 2.5%, more especially from 0.1 to 1 %, of ANTOX; the percentages are weight percent based on the weight of COMP.

Specific embodiments of COMP comprise, for example, 0.1 or 10% of POLSORB; the percentages are weight of POLSORB/volume of COMP.
Preferably, COMP comprises from 0.00001 to 10%, more preferably from 0.00002 to 1%, even more preferably from 0.0001 to 0.5%, especially from 0.0001 to 0.3%, of ANTOX; the percentages are weight of ANTOX/volume of COMP.
Specific embodiments of COMP comprise, for example, 0.0002 or 0.02% of ANTOX; the percentages are weight of ANTOX/volume of COMP.

Preferably, the ratio of the weight of POLSORB to the weight of ANTOX in COMP is at least 50:1, more preferably at least 100:1, even more preferably at least 250:1, especially at least 400:1.
In specific embodiments of COMP the ratio of the weight of POLSORB to the weight of ANTOX in COMP is 500:1.
Preferably, the ratio of the weight of POLSORB to the weight of ANTOX in the compositions of the invention is from 50:1 to 1000:1, more preferably from 100:1 to 900:1, even more preferably from 250:1 to 750:1, especially from 400:1 to 600:1.

Another subject of the invention is a method for preparation of COMP by mixing POLSORB with ANTOX; with COMP, POLSORB and ANTOX as defined herein also with all their embodiments.
The mixing can be done by any conventional means known to the skilled person in the art. Devices which can be used for the mixing are also known to the skilled person such as mixers or blenders. The mixing can also be done by stirring the components of COMP. POLSORB and ANTOX can be mixed in any convenient physical state, such as liquid or solid state. The mixing can be done in any order.

If COMP comprises further components in addition to POLSORB and ANTOX, COMP can be prepared by mixing all the components of COMP together. The mixing of the components can be done in any order.
The components of COMP can be used for the preparation of COMP, in any physical state, preferably in liquid or solid state, for example, in lyophilized state, dried state or as aqueous solutions or aqueous or non-aqueous suspensions.

Another subject of the invention is a method for reducing the oxidative degradation of POLSORB by bringing ANTOX into contact with POLSORB; with ANTOX and POLSORB as defined herein, also with all their embodiments.
Another subject of the invention is the use of ANTOX to reduce the oxidative degradation of POLSORB, preferably by bringing ANTOX into contact with POLSORB; with ANTOX and POLSORB as defined herein, also with all their embodiments.
ANTOX can be brought into contact with POLSORB by any means known to the skilled person in the art, such as for example by mixing the two components.
When ANTOX is mixed with POLSORB, POLSORB can be used for the mixing in the form of a composition comprising POLSORB and FURTHCOMP.

According to the methods and uses of the invention, the reduction of the oxidative degradation of POLSORB by bringing ANTOX into contact with POLSORB, providing COMP, is for example, at least 5%, preferably at least 10%, more preferably at least 20%; the percent are weight of POLSORB/volume of COMP. Without a contact of ANTOX with POLSORB one can observe oxidative degradation of POLSORB of over 20%.
Oxidative degradation can indirectly also be observed by the formation of FFA. According to the methods and uses of the invention, the reduction of the oxidative degradation of POLSORB by bringing ANTOX into contact with POLSORB, providing COMP, reduces the formation of FFA. The reduction can be for example by at least 1%, preferably at least 2%, more preferably at least 5%, even more preferably at least 10%, compared to a composition comprising POLSORB but no ANTOX; the percent are based on ppb of COMP. FFA which are formed by oxidative degradation of POLSORB can be lauric acid, myristic acid, palmitic acid, stearic acid, or oleic acid.

A typical oxidative substance which induces oxidative degradation of POLSORB is air. ANTOX can for example be used to reduce the degradative effect of air on POLSORB.

Preferably, the mixing of ANTOX with PS20 reduces the formation of at least one of lauric acid, myristic acid and palmitic acid compared to PS20 which was not mixed with ANTOX.
Preferably, the mixing of ANTOX with PS80 reduces the formation of at least one of palmitic acid, stearic acid and oleic acid compared to PS80 which was not mixed with ANTOX.
HPLC-FMA can be used to measure the concentration of POLSORB, such as demonstrated in the Examples.
LC-UV-MS can be used to quantify FFA.

Typical temperatures where COMP needs to be stabilized against oxidative degradation of POLSORB are from -80°C to 70°C, preferably from -40°C to 60°C, more preferably from -20°C to 50°C, and even more preferably from -5°C to 45°C.
Typical timeframes where COMP needs to be stabilized against oxidative degradation of POLSORB are for at least 1 week, for at least 2 weeks, for at least 3 weeks, for at least 4 weeks, for at least 5 weeks, for at least 6 weeks, for at least 7 weeks, for at least 2 months, for at least 6 months, or for at least 1 year.
In one embodiment, COMP shows reduced degradation of POLSORB or a reduction in the formation of FFA for at least 6 weeks at a temperature from -5°C to 45°C, preferably for at least 2 months from -5°C to 45°C, more preferably for at least 6 months from -5°C to 45°C, for example if POLSORB is exposed to air.
Oxidative degradation can indirectly also be observed by the formation of peroxide, 4HNE or hexanal. According to the methods and uses of the invention, the mixing of ANTOX with POLSORB, providing COMP, reduces the formation of peroxide, 4HNE or hexanal. The reduction can be for example by at least 1%, preferably at least 2%, more preferably at least 5%, even more preferably at least 10%, especially at least 20%, more especially at least 30%, even more especially at least 50%, in particular at least 70%, compared to a composition comprising POLSORB but no ANTOX; the percent are based on ppb of COMP.
FOX assay can for example be used to quantify peroxide levels. LC-UV-MS can for example be used to quantify 4HNE levels. GC-MS can for example be used to quantify hexanal levels.

Oxidative degradation can indirectly also be observed by the formation of headspace degradants heptanone, octanoic acid methyl ester, or hexadecanoic acid 14-methyl-methyl ester. According to the methods and uses of the invention, the mixing of ANTOX with POLSORB, especially of PS20, providing COMP, reduces the formation of said headspace degradants. The reduction can be for example by at least 1%, preferably at least 2%, more preferably at least 5%, even more preferably at least 10%, especially at least 20%, more especially at least 30%, even more especially at least 50%, in particular at least 70%, compared to a composition comprising POLSORB but no ANTOX; the percent are based on ppb of COMP.
Oxidative degradation can indirectly also be observed by the formation of headspace degradants 9-octadecen-12-ynoic acid methyl ester, pentanal, 1-pentanol, hexanol, 2-heptanone, 2-heptanal, octanal, 2-octenal, nonanal, 2-nonenal, and 2-decenal. According to the methods and uses of the invention, the mixing of ANTOX with POLSORB, especially of PS80, providing COMP, reduces the formation of said headspace degradants. The reduction can be for example by at least 1% preferably at least 2%, more preferably at least 5%, even more preferably at least 10%, especially at least 20%, more especially at least 30%, even more especially at least 50%, in particular at least 70%, compared to a composition comprising POLSORB but no ANTOX; the percent are based on ppb of COMP.

The definitions of the words or drawing elements described herein are meant to include not only the combination of elements which are literally set forth, but all equivalent structure, material or acts for performing substantially the same function in substantially the same way to obtain substantially the same result. In this sense, it is therefore contemplated that an equivalent substitution of two or more elements may be made for any one of the elements described and its various embodiments or that a single element may be substituted for two or more elements in a claim.

Changes from the claimed subject matter as viewed by a person with ordinary skill in the art, now known or later devised, are expressly contemplated as being equivalents within the scope intended and its various embodiments. Therefore, obvious substitutions now or later known to one with ordinary skill in the art are defined to be within the scope of the defined elements. This disclosure is thus meant to be understood to include what is specifically illustrated and described herein, what is conceptually equivalent, what can be obviously substituted, and also what incorporates the essential ideas.

### EXAMPLES

### Sample preparation

Studies were performed to investigate oxidative degradation of PS20 and PS80 in aqueous solutions containing the antioxidants BHT or BHA. The following solutions were tested:
1) polysorbate solutions of 10% (w/v) ± 0.02% (w/v) of the respective antioxidant,
2) polysorbate solutions of 0.1% (w/v) ± 0.0002% (w/v) of the respective antioxidant.
For solution 1), stabilized (containing the antioxidant BHT or BHA at a concentration of 0.2% (w/w)) and unstabilized (not containing any antioxidant) neat PS was diluted to a concentration of 10% (w/v) by adding 5 g of PS into a 50 mL volumetric flask and filling up to the 50 mL mark with H₂O. The solution was stirred with a magnetic stirrer for about 20 minutes to ensure complete dissolution of PS. 5 ml of each solution was aliquoted into 6R vials. Each vial was closed with a stopper and a cap.

For solution 2), 50 micro L of the 10% (w/v) PS solutions were transferred into 6R vials and filled up with 4.95 mL H₂O to a final concentration of 0.1% (w/v) PS.

These solutions were used in Examples 1 to 5.

### Storage conditions

Throughout the experiments, the solutions were subjected to three different conditions as follows:
- **"T0":**: stored at -20°C for 7 weeks,
- **"H₂O₂":**: stored at 25°C and 60% humidity for 7 weeks in the presence of hydrogen peroxide (H₂O₂) at a concentration of 10% (w/w) based on the molar ratio of used antioxidant.
- **"Air":**: stored at 40°C and 75% humidity for 7 weeks and exposed to air (needle in vial stopper for free air exchange during incubation).

Humidity in case of "Air" was set at a higher value compared to "H₂O₂" in order to reduce evaporation of water at the respective higher temperature.

These storage conditions were used in Examples 1 to 5.

### Example 1: BHT and BHA reduce peroxide levels in polysorbate-containing solutions

A FOX assay was used to quantify peroxide levels in solutions of 10% PS (w/v) ± 0.02% (w/v) of the antioxidants BHT or BHA.

Quantification of peroxides was performed according to instructions of 'PIERCE Quantitative Peroxide Assay Kits' from Thermo Scientific.

The assay kit consists of two reagent solutions. The first reagent solution contains 25 mM ammonium ferrous (II) sulfate and 2.5 M sulfuric acid. The second is composed of 100 mM sorbitol and 125 micro M xylenol orange in water. Both reagent solutions are mixed in a ratio of 1:100 (first: second reagent solution) prior to use to obtain the working reagent (WR).

H₂O₂ was used to prepare standard curves from 5 micro M to 100 micro M H₂O₂ in H₂O. Hence, the levels of peroxides in PS samples are calibrated to H₂O₂ standards.

Of each sample and standard 20 micro L were pipetted into a well of a 96 well EIA/RIA plate from Costar. Then 200 micro L of the WR was carefully added to the samples in the wells and incubated for 20 minutes at ambient temperature. Afterwards the samples were directly analyzed by SpectraMax® M2 plate reader at an absorbance wavelength of 595 nm. 595nm were used instead of the actual absorbance maximum at 560 nm because it was recommended as the optimal absorbance wavelength for analysis performed by the plate reader in the protocol of Thermo Scientific.

By introducing the WR to peroxides present in the sample or standard, sorbitol forms immediately a peroxyl radical as an intermediate product. The peroxyl radicals quantitatively oxidize ferrous (Fe²⁺) to ferric (Fe³⁺) ions. The formed ferric ions react with xylenol orange to a complex, which has an absorbance maximum at 560nm. The peroxide level present in the samples was determined from the calibration curve of H₂O₂ in H₂O.

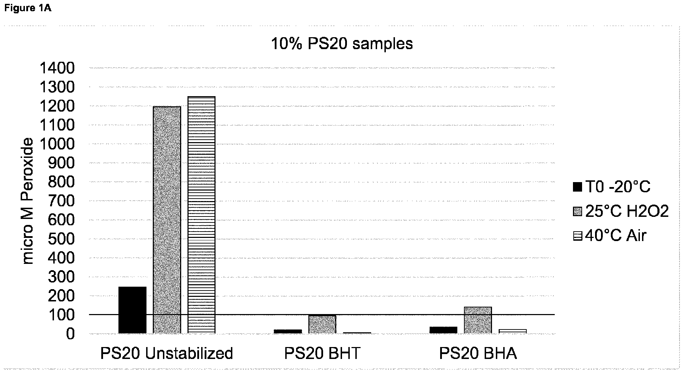

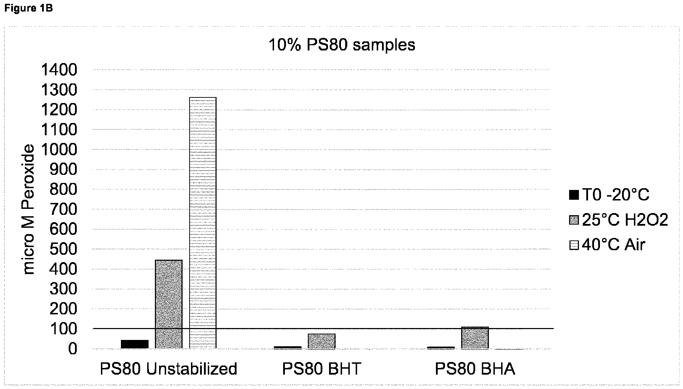

The reporting limit (RL) was 10 micro M. Note: 10% PS-containing samples were diluted to concentration of 1% prior to analysis and measured values were multiplied by 10 to obtain calculated values of peroxide for the 10% samples. Figures 1A and 1B plot the calculated values.

In Table 1, calculated peroxide concentrations in the samples are summarized:

**Table 1**

| **PS sample** | **ANTOX** | **Incubation condition** | **Absorbance at 595nm** | **Calculated Concentration in micro M** |
|---|---|---|---|---|
| PS20 - 10% | - | T0 | 0.768 | 246 |
| PS20 - 10% | - | H₂O₂ | 2.276 | 1197 |
| PS20 - 10% | - | Air | 2.361 | 1250 |
| PS20 - 10% | BHT | T0 | 0.412 | 22* |
| PS20 - 10% | BHT | H₂O₂ | 0.531 | 97* |
| PS20 - 10% | BHT | Air | 0.385 | 5* |
| PS20 - 10% | BHA | T0 | 0.434 | 36* |
| PS20 - 10% | BHA | H₂O₂ | 0.603 | 110 |
| PS20 - 10% | BHA | Air | 0.413 | 23* |
| PS80 - 10% | - | T0 | 0.445 | 43* |
| PS80 - 10% | - | H₂O₂ | 1.083 | 445 |
| PS80 - 10% | - | Air | 2.83 | 1250 |
| PS80 - 10% | BHT | T0 | 0.395 | 11* |
| PS80 - 10% | BHT | H₂O₂ | 0.497 | 76* |
| PS80 - 10% | BHT | Air | 0.361 | < RL* |
| PS80 - 10% | BHA | T0 | 0.393 | 10* |
| PS80 - 10% | BHA | H₂O₂ | 0.552 | 110 |
| PS80 - 10% | BHA | Air | 0.376 | < RL* |

| | | | | |
|---|---|---|---|---|
| *Values below RL. | | | | |

As can be seen from Table 1 and from Figure 1A, the "PS20 unstabilized" solution produced only a low amount of peroxide under the T0 condition (246 micro M). Under H₂O₂ and Air conditions, high amounts (> 1000 micro M) of peroxide were produced when PS20 solution was unstabilized. For the "PS20 BHT" and "PS20 BHA" solutions, the addition of BHT or BHA resulted in a strong reduction of peroxide levels under both H₂O₂ and Air conditions to well below the RL corresponding to 100 micro M (thick line in Figure 1A). As can be seen from Table 1 and from Figure 1B, a very similar result was obtained for the "PS80 unstabilized" solution compared to the "PS80 BHT" and "PS80 BHA" solutions.

In a separate experiment, it was confirmed that the BHT did not act as a reducing agent to interfere with the FOX assay (data not shown).

### Example 2: BHT and BHA reduce 4-HNE levels in polysorbate 80-containing solutions

LC-UV-MS was used to quantify 4HNE levels in solutions of 10% PS80 (w/v) ± 0.02% (w/v) of the antioxidants BHT or BHA.

The following LC-UV-MS conditions were applied:
Column: Phenomenex, Luna 3u C(8)2 100 A, 50 x 2.00 mm, 40°C; injection volume: 25 micro L; flow: 400 micro L/min; gradient: 5% B for 1 min, then 5% B to 100% B within 7 min, then 4 min at 100%, at the end 3 min at 5% B (A: 10 mM aqueous ammonium formate, B: ACN); UV at 220 nm, MS: ESI positive or negative, m/z = 100 - 2000.

Quantification of 4HNE was achieved by integration of the LC-UV signals (lambda = 220 nm) and using a calibration curve obtained from the integrated LC-UV signals (lambda = 220 nm) of the external reference 4HNE. As a reference standard aqueous 4HNE solutions with concentrations between 0.05 ppm and 1 ppm were prepared and directly analyzed by LC-UV-MS. 10% (w/v) PS samples were diluted to a final PS concentration of 0.1% (w/v) prior to analysis.

The RL was 50 micro M 4HNE.

In Table 2, 4HNE concentrations in the samples are summarized:

**Table 2**

| **PS Sample** | **ANTOX** | **Incubation condition** | **Y = Area** | **X = measured concentration in ppb (100- fold diluted)** | **X = calculated concentration in ppb** |
|---|---|---|---|---|---|
| PS80 - 10% | - | T0 | 4413 | 17* | 1700* |
| PS80 - 10% | - | Air | 15926 | 61 | 6068 |
| PS80 - 10% | BHT | T0 | 4272 | 16* | 1647* |
| PS80 - 10% | BHT | Air | 4347 | 17* | 1675* |
| PS80 - 10% | BHA | T0 | 4683 | 18* | 1802* |
| PS80 - 10% | BHA | Air | 5262 | 20* | 2022* |

| | | | | | |
|---|---|---|---|---|---|
| *Values below RL. | | | | | |

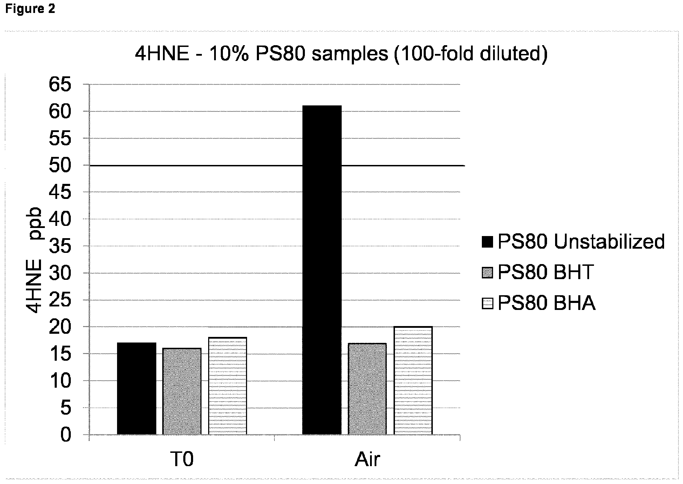

Figure 2 plots the data for the measured concentrations of 4HNE (samples were 100-fold diluted). Table 2 and Figure 2 show that 61 micro M 4HNE was measured for the "PS80 unstabilized" solution under the Air condition. In PS80 solutions additionally containing BHT or BHA under the Air condition, the amount of 4HNE was reduced to well below the RL (thick line at 50 micro M in Figure 2B).

### Example 3: PS20 and PS80 are stabilized in the presence of BHT and BHA

HPLC FMA was used to measure the concentration of polysorbate in solutions of 10% PS20 ± 0.02% (w/v) of the antioxidants BHT or BHA, solutions of 10% PS80 (w/v) ± 0.02% (w/v) of the antioxidants BHT or BHA, and solutions of 0.1% PS80 (w/v) ± 0.0002% (w/v) of the antioxidants BHT or BHA. Samples were diluted to the target concentration of 0.04% PS (w/v) prior to analysis.

The following HPLC fluorescence detector conditions were applied:
Column: knitted reaction coil L x I.D. 20mx 0.25mm, volume 1.0mL SUPELCO, ambient temperature; injection volume: 10 micro L; flow: 1.5 mL/min; isocratic 2min (A: 0.15M sodium chloride, 0.05 M L-histidine, pH = 6, 5%(v/v) acetonitrile, 5 micro M N-phenyl-1-naphtylamine (NPN), 15 ppm Brij® 35); FLD: lambda excitation = 350 nm, lambda emission = 420 nm, data mode: emission.

Quantification of each sample type was performed by using 10% T0 samples for preparation of an external calibration curve from 0.02% to 0.06% PS.

Empower 2 was used as software for quantification.

The assay is based on the uptake of the fluorescent dye NPN by surfactant micelles using flow injection analysis and subsequent fluorescence detection. The dye is present in the mobile phase. In the lipophilic surrounding in the inside of the micelles the fluorescent signal is 10-fold higher than in aqueous mobile phase. Therefore, the concentration of polysorbate can be measured according to the formation of micelles and the uptake of the dye by the subsequent increase of the signal intensity.

In Table 3, PS concentrations in the samples are summarized:

**Table 3**

| **PS sample** | **ANTOX** | **Incubation condition** | **Y = Areas of two injections** | **X= calculated concentration in % of the average of 2 injections** |
|---|---|---|---|---|
| PS20 - 10% | - | T0 | 827433155 | 0.03971 |
| | | | 857169672 | |
| PS20 - 10% | - | Air | 583583793 | 0.02908 |
| | | | 588908605 | |
| PS20 - 10% | BHT | T0 | 864035519 | 0.03960 |
| | | | 844509462 | |
| PS20 - 10% | BHT | Air | 858881494 | 0.03962 |
| | | | 850515345 | |
| PS20 - 10% | BHA | T0 | 850876867 | 0.03936 |
| | | | 846897856 | |
| PS20 - 10% | BHA | Air | 833020308 | 0.03872 |
| | | | 832611298 | |
| PS80 - 10% | - | T0 | 1995997721 | 0.04141 |
| | | | 1988105750 | |
| PS80 - 10% | - | Air | 740557015 | 0.01591* |
| | | | 745558561 | |
| PS80 - 0.1% | - | T0 | 1841230412 | 0.03839 |
| | | | 1847168423 | |
| PS80 - 0.1% | - | H₂O₂ | 773769354 | 0.01659* |
| | | | 778400281 | |
| PS80 - 10% | BHT | T0 | 1960420604 | 0.04148 |
| | | | 1941766115 | |
| PS80 - 10% | BHT | Air | 1930575380 | 0.04117 |
| | | | 1940625718 | |
| PS80 - 0.1% | BHT | T0 | 1923177230 | 0.04118 |
| | | | 1949062840 | |
| PS80 - 0.1% | BHT | H₂O₂ | 2014216558 | 0.04279 |
| | | | 2017087491 | |
| PS80 - 10% | BHA | T0 | 1988819413 | 0.04014 |
| | | | 1967397092 | |
| PS80 - 10% | BHA | Air | 1919356388 | 0.03908 |
| | | | 1922946492 | |
| PS80 - 0.1% | BHA | T0 | 1907783571 | 0.03886 |
| | | | 1910740286 | |
| PS80 - 0.1% | BHA | H₂O₂ | 1903980415 | 0.03873 |
| | | | 1900283571 | |

| | | | | |
|---|---|---|---|---|
| *Values below RL given by the lowest standard of the calibration curve (0.02%). | | | | |

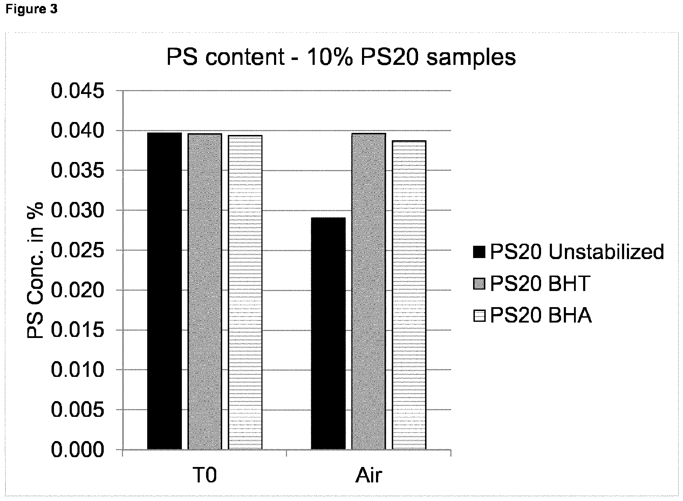

As can be seen from Table 3 and from Figure 3, the "10% PS20 unstabilized" Air sample showed a reduced PS concentration - the reduction is around 25% from the target concentration of 0.04% PS (w/v). In 10% PS20 solutions containing BHT or BHA in addition, the concentration of PS20 in the Air samples was restored to a concentration approximating the target concentration of 0.04% PS (w/v).

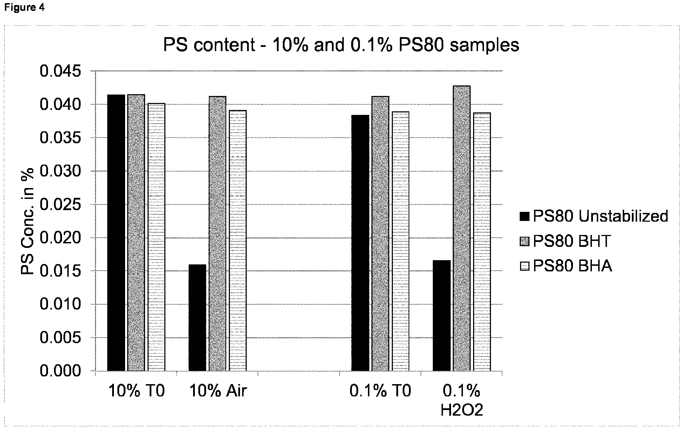

As can be seen from Table 3 and from Figure 4, left side, a similar result was obtained under the Air condition for the "10% PS80 unstabilized" solution compared to the "PS80 BHT" and "PS80 BHA" solutions. However, the effect was more pronounced as the "10% PS80 unstabilized" Air sample shows a PS concentration around 50% less than the target concentration of 0.04% PS (w/v). In 10% PS80 solutions containing BHT or BHA in addition, the concentration of PS80 in the air samples was restored to a concentration approximating the target concentration of 0.04% PS (w/v).

Figure 4, right side, also shows that the "0.1% PS80 unstabilized" sample under the H₂O₂ condition showed a greatly reduced PS concentration as the reduction is around 50% from the target concentration of 0.04% PS (w/v). In 0.1% PS80 solutions containing BHT or BHA in addition, the concentration of PS80 in the H₂O₂ samples was restored to a concentration approximating the target concentration of 0.04% PS (w/v).

### Example 4: BHT and BHA reduce the formation of free fatty acids in polysorbate-containing solutions

LC-UV-MS was used to quantify FFA levels in solutions of 10% PS (w/v) ± 0.02% (w/v) of the antioxidants BHT or BHA. The FFAs lauric acid, myristic acid and palmitic acid were quantified for PS20-containing solutions and palmitic acid, stearic acid and oleic acid were quantified for PS80-containing solutions. The RL for all FFAs was 50000 ppb.

LC-UV-MS conditions were applied as in Example 2. Quantification of the FFAs was achieved by integration of the LC-MS signals at their corresponding ion trace (lauric acid = m/z 199.3; myristic acid = m/z 227.3; palmitic acid = m/z 255.3; stearic acid = m/z 283.3; oleic acid = m/z 281.3) in negative mode and using a calibration curve obtained from the integrated LC-MS signals of each corresponding ion trace of the external reference of lauric acid, myrisitic acid, palmitic acid, stearic acid and oleic acid. FFA reference standards in aqueous solutions at concentrations from 0.5 ppm to 5 ppm were prepared. All reference standard solutions were directly analyzed by LC-UV-MS.

In Table 4, FFA concentrations in the samples are summarized:

**Table 4**

| **Fatty acid** | **PS Sample** | **ANTOX** | **Incubation condition** | **Y = Area** | **X = calculated fatty acid concentration in ppb** |
|---|---|---|---|---|---|
| **Lauric acid** | PS20 - 10% | - | T0 | 340076 | 116398 |
| | PS20 - 10% | - | Air | 2537670 | 743099 |
| RL 50000ppb | PS20 - 10% | BHT | T0 | 410786 | 136563 |
| | PS20 - 10% | BHT | Air | 484897 | 157697 |
| | PS20 - 10% | BHA | T0 | 344937 | 117784 |
| | PS20 - 10% | BHA | Air | 596732 | 189590 |
| **Myristic acid** | PS20 - 10% | - | T0 | 268718 | 23739* |
| | PS20 - 10% | - | Air | 1865690 | 407140 |
| RL 50000ppb | PS20 - 10% | BHT | T0 | 280992 | 26686* |
| | PS20 - 10% | BHT | Air | 419218 | 59871 |
| | PS20 - 10% | BHA | T0 | 301015 | 31493* |
| | PS20 - 10% | BHA | Air | 448067 | 66797 |
| **Palmitic acid** | PS20 - 10% | - | T0 | 352953 | 16492* |
| | PS20 - 10% | - | Air | 2080571 | 168918 |
| RL 50000ppb | PS20 - 10% | BHT | T0 | 361028 | 17205* |
| | PS20 - 10% | BHT | Air | 469379 | 26765* |
| | PS20 - 10% | BHA | T0 | 358247 | 16960* |
| | PS20 - 10% | BHA | Air | 660095 | 43591* |
| **Palmitic acid** | PS80 - 10% | - | T0 | 126074 | N/A |
| | PS80 - 10% | - | Air | 937434 | 68060 |
| RL 50000ppb | PS80 - 10% | BHT | T0 | 11986 | N/A |
| | PS80 - 10% | BHT | Air | 187354 | 1882* |
| | PS80 - 10% | BHA | T0 | 119155 | N/A |
| | PS80 - 10% | BHA | Air | 288362 | 10794* |
| **Stearic acid** | PS80 - 10% | - | T0 | 42067 | 57294 |
| | PS80 - 10% | - | Air | 255619 | 66863 |
| RL 50000ppb | PS80 - 10% | BHT | T0 | 44328 | 57395 |
| | PS80 - 10% | BHT | Air | 70846 | 58583 |
| | PS80 - 10% | BHA | T0 | 45216 | 57435 |
| | PS80 - 10% | BHA | Air | 112782 | 60463 |
| **Oleic acid** | PS80 - 10% | - | T0 | 2502492 | 47867* |
| RL 50000ppb | PS80 - 10% | - | Air | 23598216 | 531429 |
| | PS80 - 10% | BHT | T0 | 2495437 | 47705* |
| | PS80 - 10% | BHT | Air | 5205282 | 109821 |
| | PS80 - 10% | BHA | T0 | 2506994 | 47970* |
| | PS80 - 10% | BHA | Air | 9557696 | 209588 |

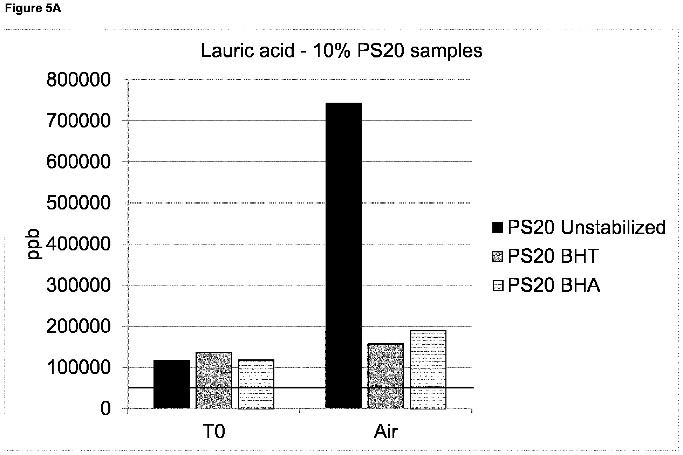

Table 4 and Figure 5A show that the unstabilized 10% PS20 solution under the Air condition produced lauric acid at around 700000 ppb. The addition of BHT or BHA resulted in a strong reduction of lauric acid levels to below 200000 ppb. Lauric acid levels were rather similar at around 100000 ppb under the T0 condition.

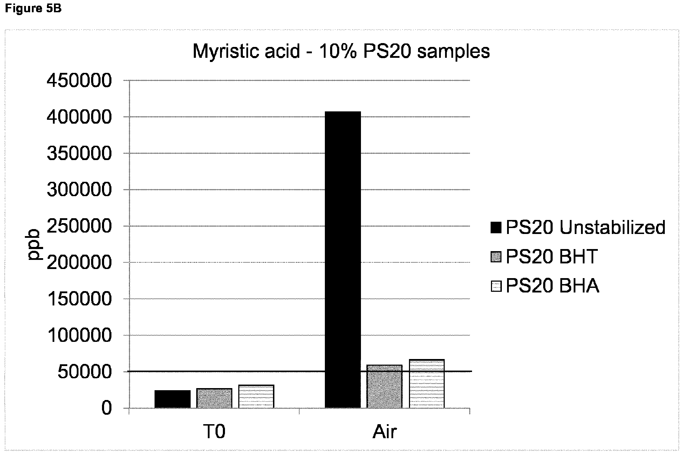

Table 4 and Figure 5B show that the unstabilized 10% PS20 solution under the Air condition produced myristic acid at around 400000 ppb. The addition of BHT or BHA resulted in a strong reduction of myristic acid levels to around 50000 ppb. Myristic acid levels under the T0 condition were under the RL.

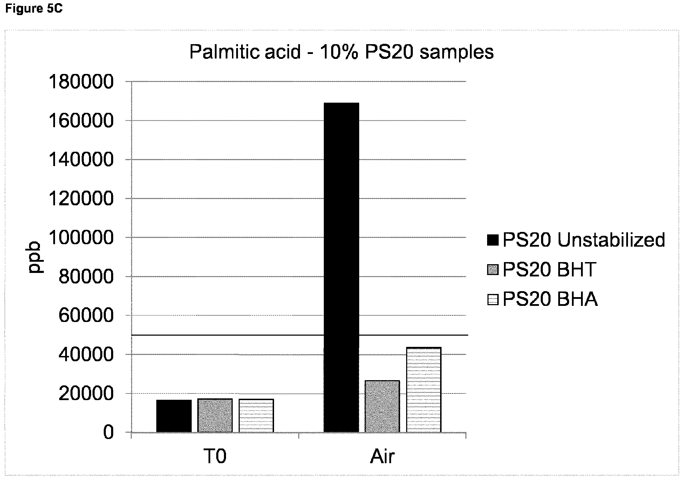

Table 4 and Figure 5C show that the unstabilized 10% PS20 solution under the Air condition produced palmitic acid at around 170000 ppb. The addition of BHT or BHA resulted in a strong reduction of palmitic acid levels to below the RL. Palmitic acid levels under the T0 condition were under the RL.

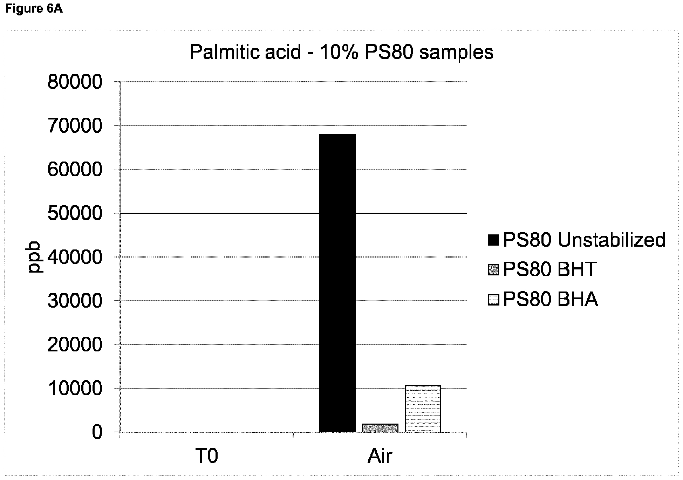

Table 4 and Figure 6A show that the unstabilized 10% PS80 solution under the Air condition produced palmitic acid at almost 70000 ppb. The addition of BHT or BHA resulted in a strong reduction of palmitic acid levels below the RL. No palmitic acid could be measured under the T0 condition.

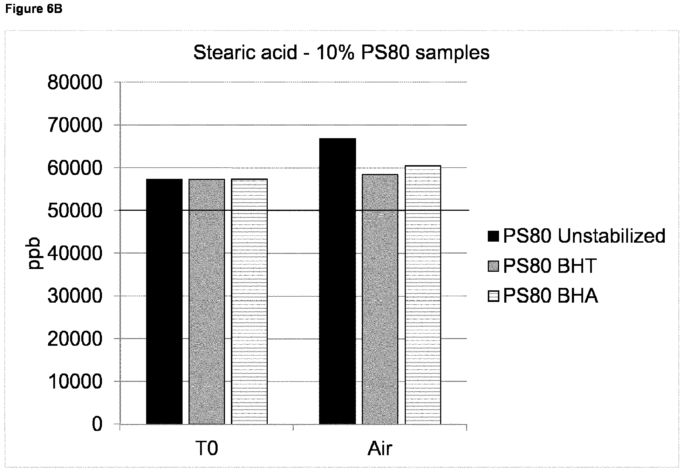

Table 4 and Figure 6B show that the unstabilized 10% PS80 solution under the Air condition produced stearic acid at around 65000 ppb. The addition of BHT or BHA resulted in a reduction of myristic acid levels to around 60000 ppb in each case. Stearic acid levels were rather similar at around 58000 ppb under the T0 condition.

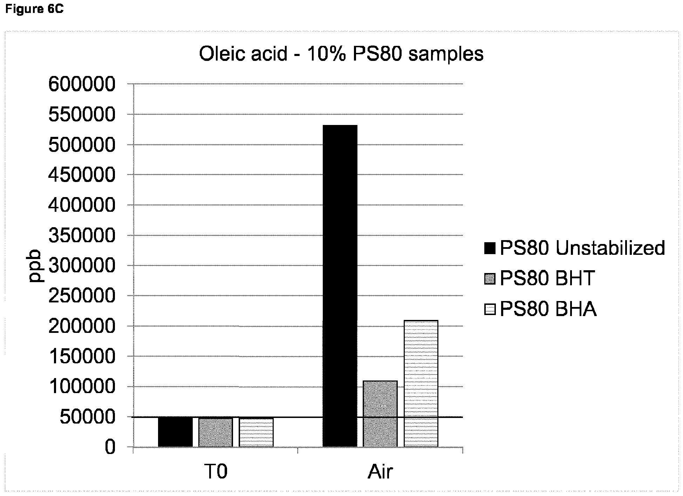

Table 4 and Figure 6C show that the unstabilized 10% PS80 solution under the Air condition produced oleic acid at around 525000 ppb. The addition of BHT or BHA resulted in a strong reduction of oleic acid levels to around 100000 ppb for BHT and around 200000 ppb for BHA. Oleic acid levels were rather similar under the T0 condition.

### Example 5: BHT and BHA reduce the formation of hexanal in polysorbate-containing solutions

GC-MS was used to quantify headspace hexanal levels for solutions of 10% PS (w/v) ± 0.02% (w/v) of the antioxidants BHT or BHA.

The following HS-GC-MS conditions were applied:
Headspace oven: 10 min at 80°C, Column: Agilent HP-5MS, 30 m x 250 micro M x 0.25 micro M; GC oven: 3 min at 35°C, then 20°C/min to 320°C, then 10 min at 320°C; column flow: 1.3 mL/min; MS: Full scan, m/z = 35 - 300. As a reference standard aqueous toluene solutions with concentrations between 0.1 ppm and 1 ppm in 10 mL H₂O were prepared and directly analyzed by HS-GC-MS.

1 mL of each sample was transferred into a headspace vial with a volume of 20 mL, closed, and analyzed directly.

Quantification was achieved by integration of the HS-GC-MS signals (TIC) and using a calibration curve obtained from the integrated HS-GC-MS signals (TIC) of the external reference toluene.

Tentative identification of degradants was performed by comparing the mass spectrum of each GC-MS signal against the NIST14 standard reference database.

The degradant most consistently identified by HS-GC-MS to show a value above the RL of 100 ppb was hexanal. The following results in Table 5 were obtained for hexanal under the various conditions:

**Table 5:**

| 10% PS80 unstabilized | 10% PS80 unstabilized | 10% PS80 unstabilized | 10% PS80 BHT | 10% PS80 BHT | 10% PS80 BHT | 10% PS80 BHA | 10% PS80 BHA | 10% PS80 BHA |
|---|---|---|---|---|---|---|---|---|
| T0 | H₂O₂ | Air | T0 | H₂O₂ | Air | T0 | H₂O₂ | Air |
| 20 | 81 | 137 | < RL | < RL | < RL | < RL | < RL | < RL |

The 10% PS80 aqueous solution under the Air condition produced hexanal at 137 ppb. Hexanal levels were below the RL for the T0 and H₂O₂ conditions. When BHT or BHA were added to 10% PS80 aqueous solutions, no hexanal could be detected under the T0, H₂O₂ or Air conditions.

## Claims

1. A composition COMP comprising a polysorbate POLSORB and an antioxidant ANTOX selected from the group consisting of butylated hydroxytoluene, butylated hydroxyanisole, and mixtures thereof.

2. Composition according to claim 1, wherein POLSORB is selected from the group consisting of polysorbate 20, polysorbate 21, polysorbate 40, polysorbate 60, polysorbate 61, polysorbate 65, polysorbate 80, polysorbate 81, polysorbate 85, and polysorbate 120, and mixtures thereof.

3. Composition according to claim 1 or claim 2, wherein COMP comprises from 0.001 to 99.99999% of POLSORB; the percentages are weight of POLSORB/volume of COMP.

4. Composition according to one or more of claims 1 to 3, wherein COMP comprises from 0.00001 to 20% of ANTOX; the percentages are weight percent based on the weight of COMP.

5. Composition according to one or more of claims 1 to 4, wherein the ratio of the weight of POLSORB to the weight of ANTOX in COMP is at least 50:1.

6. Method for preparation of COMP by mixing POLSORB with ANTOX; with COMP, POLSORB and ANTOX as defined in claim 1.

7. Use of ANTOX to reduce the oxidative degradation of POLSORB, preferably by bringing ANTOX into contact with POLSORB; with ANTOX and POLSORB as defined in claim 1.

8. Use of ANTOX according to claim 7, wherein the reduction of the oxidative degradation of POLSORB by bringing ANTOX into contact with POLSORB, providing COMP, is at least 5%; the percent are weight of POLSORB/volume of COMP.

9. Use of ANTOX according to claim 7, wherein the reduction of the oxidative degradation of POLSORB by bringing ANTOX into contact with POLSORB, providing COMP, reduces the formation of FFA by at least 1%, the percent are based on ppb of COMP.

10. Use of ANTOX according to one or more of claims 7 to 9, wherein the oxidative substance which induces oxidative degradation of POLSORB is air.
